# EUROPEAN PATENT APPLICATION

(11) **EP 4 368 107 A1**
(43) Date of publication of application: **15.05.2024**
(21) Application number: 22858722.6
(22) Date of filing: 16.08.2022
(51) Int. Cl.: A61B 5/346, A61B 5/327, A61B 5/308, A61B 5/00, G16H 50/50, G16H 50/20, G16H 50/70

(54) **HEALTH CONDITION PREDICTION SYSTEM USING ASYNCHRONOUS ELECTROCARDIOGRAM**

(30) Priority: 17.08.2021 KR 20210107769
(71) Applicant: Medicalai Co., Ltd., Seoul 06302 (KR)
(72) Inventor: KWON, Joon Myoung, Seoul 06629 (KR)
(74) Representative: Isarpatent
(86) International application number: PCT/KR2022/012216
(87) International publication number: WO 2023/022485

(57) **Abstract**

Disclosed herein is a health condition prediction system using an asynchronous electrocardiogram, including: a single-lead electrocardiogram measurement unit provided with two electrodes, and configured to obtain asynchronous electrocardiogram data by measuring electrocardiograms for at least two electrical axes at different times; and a prediction unit configured to predict the presence/absence of a disease and the degree of the disease from the asynchronous electrocardiogram data through a diagnostic algorithm previously constructed by being trained on a plurality of asynchronous segment standard lead electrocardiogram datasets in which asynchronous segment standard lead electrocardiograms at different times in an asynchronous standard lead electrocardiogram segmented into the specific time units from a synchronous standard lead electrocardiogram measured for the same electrical axis and accumulated in a medical institution server are matched to the presence/absence of a disease and degree of the disease corresponding to the asynchronous segment standard lead electrocardiograms.

## Description

### Technical Field

The present invention relates to a health condition prediction system using an asynchronous electrocardiogram that can generate datasets for the training of a prediction model through the extraction of a 2- or more-lead asynchronous electrocardiogram by segmenting various types of synchronous electrocardiograms accumulated in a medical institution server and predict a disease from the 2- or more-lead asynchronous electrocardiogram data measured by a single-lead electrocardiogram device.

### Background Art

As is well known, since the development of an electrocardiogram, knowledge related to the electrocardiogram has expanded exponentially. Through electrocardiogram tests, information about the electrical functions of the heart can be obtained and various heart diseases such as arrhythmia, coronary artery disease, and myocardial disease can be diagnosed.

Recently, research into AI algorithms for electrocardiograms has been actively conducted. By AI algorithms, heart failure is detected, atrial fibrillation among arrhythmia rhythms is predicted, and gender is determined.

As described above, by overcoming human limitations, subtle changes in electrocardiogram waveforms can be detected and electrocardiogram interpretation can be further improved by AI algorithms.

Meanwhile, the electrocardiograms used in the medical field are 12-lead electrocardiograms. Such a 12-lead electrocardiogram is measured by attaching 10 electrodes, including 3 limb electrodes, 6 chest electrodes, and 1 ground electrode, and measured electrocardiogram data can be transmitted remotely.

However, it is inconvenient to expose the chest, attach 10 electrodes and then use a device in everyday life, so that a portable pad measurement device, a Galaxy Watch, an Apple Watch, or the like capable of measuring a single-lead electrocardiogram is also used.

Ae single-lead electrocardiogram measured as described above can be used to diagnose arrhythmia. However, a single-lead electrocardiogram has limitations in terms of use in the diagnosis of a disease requiring electrocardiogram information for various leads, such as myocardial infarction.

Therefore, there is a demand for a technology that can easily measure a 2- or more-lead asynchronous electrocardiogram using a single-lead electrocardiogram device or a 6-lead electrocardiogram device and predict a health condition from the measured 2- or more-lead asynchronous electrocardiogram data.

### Disclosure

### Technical Problem

A technical object to be achieved by the spirit of the present invention is to provide a health condition prediction system using an asynchronous electrocardiogram that can generate datasets for the training of a prediction model through the extraction of a 2- or more-lead asynchronous electrocardiogram by segmenting various types of synchronous electrocardiograms accumulated in a medical institution server and predict a disease from the 2- or more-lead asynchronous electrocardiogram data measured by a single-lead electrocardiogram device.

### Technical Solution

In order to accomplish the above-described object, an embodiment of the present invention provides a health condition prediction system using an asynchronous electrocardiogram, the system including: a single-lead electrocardiogram measurement unit provided with two electrodes, and configured to obtain asynchronous electrocardiogram data by measuring electrocardiograms for at least two electrical axes at different times; and a prediction unit configured to predict the presence/absence of a disease and the degree of the disease from the asynchronous electrocardiogram data, input from the single-lead electrocardiogram measurement unit and segmented into specific time units, through a diagnostic algorithm previously constructed by being trained on a plurality of asynchronous segment standard lead electrocardiogram datasets in which asynchronous segment standard lead electrocardiograms at different times in an asynchronous standard lead electrocardiogram segmented into the specific time units from a synchronous standard lead electrocardiogram measured for the same electrical axis and accumulated in a medical institution server are matched to the presence/absence of a disease and degree of the disease corresponding to the asynchronous segment standard lead electrocardiograms.

In this case, the system may further include an electrocardiogram data generation unit configured to determine the characteristics of the plurality of pieces of asynchronous electrocardiogram data measured by the single-lead electrocardiogram measurement unit, identify specific lead electrocardiogram data, and generate a plurality of pieces of remaining standard lead electrocardiogram data not corresponding to the identified specific lead electrocardiogram data; and the diagnostic algorithm may output and predict the presence/absence of the disease and the degree of the disease by using the measured asynchronous electrocardiogram data and the generated standard lead electrocardiogram data as input or using the generated standard lead electrocardiogram data as input.

Furthermore, the electrocardiogram data generation unit may generate a plurality of pieces of synchronized standard lead electrocardiogram data.

Furthermore, the diagnostic algorithm may generate the plurality of asynchronous segment standard lead electrocardiogram data sets by asynchronously extracting synchronous segment standard lead electrocardiograms obtained by segmenting standard lead electrocardiogram data, stored in a synchronized form, into the specific time units.

Furthermore, the diagnostic algorithm may be constructed to output and predict the presence/absence of a disease and degree of the disease corresponding to standard lead electrocardiogram data by using the standard lead electrocardiogram data, in which time-series information has been deleted from standard lead electrocardiogram data measured synchronously, as input.

Furthermore, the diagnostic algorithm may be trained with the individual characteristic information of an examinee reflected therein; and the prediction unit may predict the presence/absence of the disease and the degree of the disease from the asynchronous electrocardiogram data measured by the single-lead electrocardiogram measurement unit with the individual characteristic information reflected therein.

Moreover, the individual characteristic information may include demographic information including the gender and age of the examinee, basic health information including the weight, height and obesity of the examinee, disease-related information including the past history, drug history and family history of the examinee, and test information regarding a blood test, genetic test, vital signs, including blood pressure and oxygen saturation, and biosignals of the examinee.

### Advantageous Effects

According to the present invention, there are effects in that a 2- or more-lead asynchronous electrocardiogram may be easily measured utilizing a single-lead electrocardiogram device or a 6-lead electrocardiogram device and datasets for the training of a prediction model may be generated through the extraction of a 2- or more-lead asynchronous electrocardiogram by segmenting various types of synchronous electrocardiograms accumulated in a medical institution.

Furthermore, there is an effect in that a disease may be predicted from the 2- or more-lead asynchronous electrocardiogram data measured by the single-lead electrocardiogram device or the 6-lead electrocardiogram device.

Moreover, there is an effect in that multi-channel electrocardiogram data is generated from the 2- or more-lead asynchronous electrocardiogram data, so that the presence/absence of a disease and the degree of the disease can be output and predicted with higher accuracy.

### Description of Drawings

FIG. 1 is a schematic block diagram showing a health condition prediction system using an asynchronous electrocardiogram according to an embodiment of the present invention; and
FIG. 2 is a flowchart showing a prediction method performed by the health condition prediction system using an asynchronous electrocardiogram shown in FIG. 1.

### Mode for Invention

Embodiments of the present invention having the above-described features will be described in more detail below with reference to the accompanying drawings.

A health condition prediction system using an asynchronous electrocardiogram according to an embodiment of the present invention includes: a single-lead electrocardiogram measurement unit 110 provided with two electrodes, and configured to obtain asynchronous electrocardiogram data by measuring electrocardiograms for at least two electrical axes at different times; and a prediction unit 120 configured to predict the presence/absence of a disease and the degree of the disease from the asynchronous electrocardiogram data, input from the single-lead electrocardiogram measurement unit 110 and segmented into specific time units, through a diagnostic algorithm 121 previously constructed by being trained on a plurality of asynchronous segment standard lead electrocardiogram datasets in which asynchronous segment standard lead electrocardiograms at different times in an asynchronous standard lead electrocardiogram segmented into the specific time units from a synchronous standard lead electrocardiogram measured for the same electrical axis and accumulated in a medical institution server are matched to the presence/absence of a disease and degree of the disease corresponding to the asynchronous segment standard lead electrocardiograms. The health condition prediction system using an asynchronous electrocardiogram is characterized by generating datasets for the training of a prediction model through the generation of asynchronous electrocardiograms by segmenting synchronous electrocardiograms in a medical institution server and predicting a disease from the 2- or more-lead asynchronous electrocardiogram data measured by a single-lead electrocardiogram device.

The health condition prediction system using an asynchronous electrocardiogram configured as described above will be described in detail below with reference to the drawings, as follows.

First, the single-lead electrocardiogram measurement unit 110 is provided with two electrodes, obtains asynchronous electrocardiogram data by measuring electrocardiograms for at least two electrical axes at different times in such a manner as to bring the two electrodes into contact with two portions of the body of an examinee, and transmits the obtained asynchronous electrocardiogram data to the prediction unit 120 over a short-distance network.

For example, a plurality of asynchronous electrocardiograms for at least two different electrical axes may be measured by measuring a lead-I electrocardiogram corresponding to one electrical axis in such a manner as to bring the respective electrodes into contact with both hands and also measuring a lead-II electrocardiogram corresponding to another electrical axis in such a manner as to bring the respective electrodes into contact with the right and left ankles, which are different from the previous body combination for contact with the electrodes.

Furthermore, the single-lead electrocardiogram measurement unit 110 may measure asynchronous or synchronous electrocardiograms by including a wearable electrocardiogram patch 111, a smartwatch 112, or a 6-lead electrocardiogram bar for short-term measurement capable of contact or contactless electrocardiogram measurement during daily life, and may predict a corresponding health condition by additionally generating a plurality of pieces of electrocardiogram data through an electrocardiogram data generation unit 130 and inputting the plurality of pieces of electrocardiogram data to the diagnostic algorithm 121 constructed using standard lead electrocardiogram data.

In this case, the single-lead electrocardiogram measurement unit 110 may measure successive electrocardiograms of an examinee and transmit them to the prediction unit 120, or may measure two electrocardiograms at different times and transmit them to the prediction unit 120.

Next, the prediction unit 120 is a component that predicts a health condition from the segmented asynchronous electrocardiogram data of the single-lead electrocardiogram measurement unit 110 through the previously trained diagnostic algorithm 121. Through the diagnostic algorithm 121 previously constructed by being trained on a plurality of asynchronous segment standard lead electrocardiogram datasets in which the asynchronous segment standard lead electrocardiograms at different times in the asynchronous standard lead electrocardiogram segmented into specific time units from the synchronous standard lead electrocardiogram measured for the same electrical axis and accumulated in a medical institution server are matched to the presence/absence of a disease and degree of the disease corresponding to the asynchronous segment standard lead electrocardiograms, the prediction unit 120 determines the health condition of an examinee by predicting the presence/absence of a disease and the degree of the disease from the asynchronous electrocardiogram data input from the single-lead electrocardiogram measurement unit 110 and segmented into the specific time units.

In this case, the asynchronous standard lead electrocardiograms may be provided by segmenting a standard lead electrocardiogram, measured through an electrocardiogram device at a medical institution, into specific time intervals of, e.g., 2.5 seconds and obtaining the standard lead electrocardiogram segmented at different times.

Meanwhile, the diagnostic algorithm 121 may be constructed to output and predict the presence/absence of a disease and degree of the disease corresponding to the asynchronous segmented standard lead electrocardiogram data by using standard lead electrocardiogram data, to be measured asynchronously and segmented for any electrical axes, as input. Accordingly, the diagnostic algorithm 121 may predict the health condition of the examinee from asynchronous electrocardiogram data transmitted from the single-lead electrocardiogram measurement unit 110.

Alternatively, the diagnostic algorithm 121 may be constructed to output and predict the presence/absence of a disease and degree of the disease corresponding to standard lead electrocardiogram data by using the standard lead electrocardiogram data, in which time-series information has been deleted from standard lead electrocardiogram data measured synchronously for a plurality of electrical axes, as input. Accordingly, the diagnostic algorithm 121 may predict the health condition of the examinee from the asynchronous electrocardiogram data transmitted from the single-lead electrocardiogram measurement unit 110.

Alternatively, the diagnostic algorithm 121 may be constructed to convert a plurality of pieces of asynchronous electrocardiogram data, measured and input at different times from the single-lead electrocardiogram measurement unit 110, into synchronous electrocardiogram data and predict the presence/absence of a disease and the degree of the disease from the resulting synchronous electrocardiogram data.

Alternatively, the diagnostic algorithm 121 may generate a plurality of asynchronous segmented standard lead electrocardiogram data sets by asynchronously the extracting synchronous segmented standard lead electrocardiogram data obtained by segmenting the standard lead electrocardiogram data, stored in a synchronous form, into specific time intervals.

Accordingly, the diagnostic algorithm 121 may match a plurality of pieces of segmented asynchronous electrocardiogram data measured and input at different times by the single-lead electrocardiogram measurement unit 110 to standard lead electrocardiogram data which is measured asynchronously and segmented, to segmented standard lead electrocardiogram data from which time-series information has been deleted, or to segmented synchronous standard lead electrocardiogram data.

Furthermore, through the electrocardiogram data generation unit 130, the individual characteristics of the plurality of pieces of asynchronous electrocardiogram data measured by the single-lead electrocardiogram measurement unit 110 are determined, specific lead electrocardiogram data is identified, and a plurality of pieces of remaining standard lead electrocardiogram data not corresponding to the identified specific lead electrocardiogram data are generated. Accordingly, the diagnostic algorithm 121 may output and predict the presence/absence of a disease and the degree of the disease by using the asynchronous electrocardiogram data measured by the single-lead electrocardiogram measurement unit 110 and the standard lead electrocardiogram data generated by the electrocardiogram data generation 130 as input or using the standard lead electrocardiogram data generated by the electrocardiogram data generation unit 130 as input.

For example, when a plurality of pieces of standard lead electrocardiogram data are generated by the electrocardiogram data generation unit 130, individual characteristics are determined through the unique characteristics of an electrocardiogram for each lead, so that the asynchronous electrocardiogram data input from the single-lead electrocardiogram measurement unit 110 is matched to corresponding specific standard lead electrocardiogram data, and the remaining standard lead electrocardiogram data not matched to the specific standard lead electrocardiogram data is generated, with the result that a plurality of pieces of new standard lead electrocardiogram data can be generated.

Alternatively, the electrocardiogram data generation unit 130 may generate a plurality of pieces of synchronized standard lead electrocardiogram data. That is, the standard lead electrocardiogram data generated by the electrocardiogram data generation unit 130 may be an asynchronous or synchronous electrocardiogram. As described above, when the diagnostic algorithm 121 uses standard lead electrocardiogram data which is measured asynchronously or standard lead electrocardiogram data from which time-series information has been deleted, the electrocardiogram data generation unit 130 may generate asynchronous electrocardiogram data or generate electrocardiogram data without taking into consideration synchronization.

Through this, the prediction of the presence/absence of a disease and the diagnosis of the degree of the disease that need to be diagnosed using a multi-lead electrocardiogram are performed using the segmented asynchronous electrocardiogram measured by the single-lead electrocardiogram measurement unit 110. Accordingly, a disease is analyzed more accurately based on a plurality of pieces of electrocardiogram information, so that the measurement, diagnosis, examination, and prediction of a health condition can be performed.

For example, in the case of a disease that can be diagnosed using a single-lead electrocardiogram, such as arrhythmia, electrocardiograms for each beat are generated for various electrical axes by using a 2-lead electrocardiogram and a plurality of electrocardiograms generated based on it, so that the more accurate measurement, diagnosis, examination, and prediction of a health condition can be performed. In the case of a disease that can be diagnosed using a multi-lead electrocardiogram, such as myocardial infarction, myocardial infarction may be diagnosed by additionally generating a plurality of electrocardiograms.

Alternatively, although the diagnostic algorithm 121 may be a model that performs the measurement, diagnosis, examination, and prediction of a health condition using only electrocardiogram data corresponding to 2 leads to be used in standard lead electrocardiogram data for the diagnosis of a disease, it is not limited to 2 leads, but additionally generated lead electrocardiogram data may also be used, as described above.

In other words, the electrocardiogram data accumulated in a medical institution server is standard 12-lead electrocardiogram data. A standard 12-lead electrocardiogram is generated from a 2-lead electrocardiogram through the electrocardiogram data generation unit 130, and the electrocardiogram generated in this manner is input to an algorithm that performs the measurement, diagnosis, examination, and prediction of a health condition based on the standard 12-lead electrocardiogram data of the medical institution, so that more accurate prediction results can be output and a wider range of a health condition can be predicted utilizing the 2-lead electrocardiogram.

As an example, in the case of measurement by the single-lead electrocardiogram measurement unit 110, when electrocardiogram data contains a lot of noise in electrocardiogram data for one or more leads or sections or electrode contact is lost and thus measurement is not performed appropriately, the more accurate measurement, diagnosis, examination, and prediction of a health condition may be performed by generating a noise-free electrocardiogram and filling the missing electrocardiogram data with it through the electrocardiogram data generation unit 130.

Furthermore, through the single-lead electrocardiogram measurement unit 110 and the prediction unit 120, a health condition is monitored by measuring the reference electrocardiogram of an examinee in his or her usual healthy condition, and whether the electrocardiogram was measured without error and whether the health condition of the examinee is normal are predicted by comparing the electrocardiogram measured and input in real time by and from the single-lead electrocardiogram measurement unit 110 during daily life with the reference electrocardiogram. Furthermore, when an error or abnormality is predicted, warning information may be generated through a warning unit 140 and transmitted together with a beep sound through the single-lead electrocardiogram measurement unit 110 in the form of a smartwatch or a separate smart device.

More specifically, at the beginning of use, the reference electrocardiogram measured by the single-lead electrocardiogram measurement unit 110 is stored and then additional lead electrocardiograms are generated, so that a 12-lead electrocardiogram can be continuously monitored. A lead II electrocardiogram is measured by bringing the smartwatch into contact with the stomach while holding the smartwatch 112 in the right hand and stored as a reference electrocardiogram. In normal times, a lead I electrocardiogram is measured by making contact using the right hand while wearing the smartwatch 112 on the left hand, and at the same time, a plurality of lead electrocardiograms, including a lead II electrocardiogram, are generated using the lead I electrocardiogram. Accordingly, using the smartwatch 112, the measurement, diagnosis, examination, and prediction of various health conditions may be performed.

Alternatively, before the wearable electrocardiogram patch 111 is attached, a lead I electrocardiogram is measured by holding the wearable electrocardiogram patch 111 in both hands and stored as a reference electrocardiogram. Thereafter, a lead V electrocardiogram is measured by attaching the wearable electrocardiogram patch 111, and a multi-lead synchronized electrocardiogram is generated based on the continuously measured or monitored lead V electrocardiogram. Accordingly, the more accurate measurement, diagnosis, examination, and prediction of a health condition may be performed.

Furthermore, the diagnostic algorithm 121 is trained with the individual characteristic information of an examinee reflected therein, and the prediction unit 120 may predict the presence/absence of a disease and the degree of the disease from the asynchronous electrocardiogram data measured by the single-lead electrocardiogram measurement unit 110 with the individual characteristic information reflected therein.

For example, when the standard lead electrocardiogram data used when the diagnostic algorithm 121 is trained is applied, the presence/absence of a corresponding disease and the degree of the disease may be predicted with individual characteristic information reflected therein. Alternatively, standard lead electrocardiogram data is generated by the electrocardiogram data generation unit 130 with the individual characteristic information of an examinee whose electrocardiogram is measured by the single-lead electrocardiogram measurement unit 110 reflected therein, and then the presence/absence of a corresponding disease and the degree of the disease may be predicted.

In this case, the individual characteristic information may include demographic information including the gender and age of the examinee, basic health information including the weight, height and obesity of the examinee, disease-related information including the past history, drug history and family history of the examinee, and test information regarding a blood test, genetic test, vital signs, including blood pressure and oxygen saturation, and biosignals of the examinee.

Meanwhile, the prediction unit 120 may use not only a graphed electrocardiogram but also a digitized electrocardiogram as input. For example, the prediction unit 120 may further include a data conversion module 122 configured to convert asynchronous electrocardiogram data, measured by the single-lead electrocardiogram measurement unit 110, into numerical data corresponding to an electrocardiogram through a specific formula. The diagnostic algorithm 121 may predict a health condition by using numerical data corresponding to asynchronous electrocardiogram data as input.

Furthermore, the diagnostic algorithm 121 may be constructed as a variety of deep learning models such as a convolutional neural network, LSTM, an RNN, and an MLP, and may be constructed as various machine learning models such as logistic regression, a principle-based model, random forests, and a support vector machine.

For example, the diagnostic algorithm 121 may be constructed as a deep learning model using two or more pieces of electrocardiogram data measured at different times with a time difference therebetween. At least two electrocardiograms may be integrated into and input as one piece of electrocardiogram data or two or more electrocardiograms may be input to a deep learning model, the semantic features, that is, spatial time series features, measured in the midst thereof may be extracted, and then, based on this, the measurement, diagnosis, examination, and prediction of a health condition at the time when the measurement is made by the single-lead electrocardiogram measurement unit 110 or a future health condition may be performed by comparing the two electrocardiograms.

In this case, according to a method of mixing electrocardiograms measured at two or more times, a single electrocardiogram may be divided into beats, and then beats for the same lead measured at different times may be paired and input or the semantic features or result values extracted after input may be compared to each other.

Alternatively, rather than dividing an electrocardiogram measured at two or more times into beats, electrocardiogram data itself may be merged together. The electrocardiogram data measured at two or more times may be merged together and then input. The electrocardiogram data may be separated and merged for each lead and then input. The electrocardiograms measured at different times may be input to the deep learning layer of a deep learning model, semantic features or output values may be extracted, and then these may be merged to output a final conclusion. The electrocardiograms measured at different times may be separated for each lead and then input to the deep learning layer, and the features or output values extracted through deep learning may be merged at the back end to output the final conclusion.

In this case, when the electrocardiograms measured at two or more times are mixed and then used, the electrocardiograms may be input in an asynchronous state, may be synchronized on a per-bit basis, or may be synchronized based on deep learning, merged and then used.

In other words, a deep learning model may be constructed utilizing standard lead electrocardiogram data. A deep learning model that measures, diagnoses, examines, and predicts a health condition by inputting one electrocardiogram at each time is developed. Thereafter, the electrocardiograms measured at two or more times using the corresponding deep learning model are input to the deep learning model. Thereafter, results may be predicted by combining the semantic features or final output values output from the deep learning model.

As described above, as a method of combining the final output values, a variety of deep learning methods such as a convolutional neural network, LSTM, an RNN, and an MLP may be used, and various machine learning methods such as logistic regression, a principle-based model, random forests, and a support vector machine may also be used.

Through the prediction unit 120 described above, diseases of the circulatory system, endocrine, nutritional and metabolic diseases, neoplastic diseases, mental and behavioral disorders, diseases of the nervous system, diseases of the eyes and appendages, diseases of the ears and mastoids, diseases of the respiratory system, diseases of the digestive system, diseases of the skin and skin tissue, diseases of the musculoskeletal system and compressive tissue, diseases of the genitourinary system, pregnancy, childbirth and postpartum diseases, congenital anomalies, deformities, and chromosomal abnormalities may be diagnosed and predicted.

In addition, through the prediction unit 120, the damage caused by physical trauma may be identified, the prognosis thereof may be checked and the pain thereof may be measured, the risk of death or worsening attributable to trauma may be predicted, concurrent complications may be detected or predicted, and specific conditions that appear before or after birth may also be identified.

In addition, through the prediction unit 120, for the healthcare field, the health condition of an examinee, which can lead to services such as aging, sleep, weight, blood pressure, blood sugar, oxygen saturation, metabolism, stress, tension, fear, drinking, smoking, problem behavior, lung capacity, exercise volume, pain management, obesity, body mass, body composition, diet, type of exercise, lifestyle pattern recommendations, emergency management, chronic disease management, medication prescription, test recommendation, checkup recommendation, nursing, telehealth management, telemedicine, vaccination, and post-vaccination management, may be measured, diagnosed, examined, and predicted.

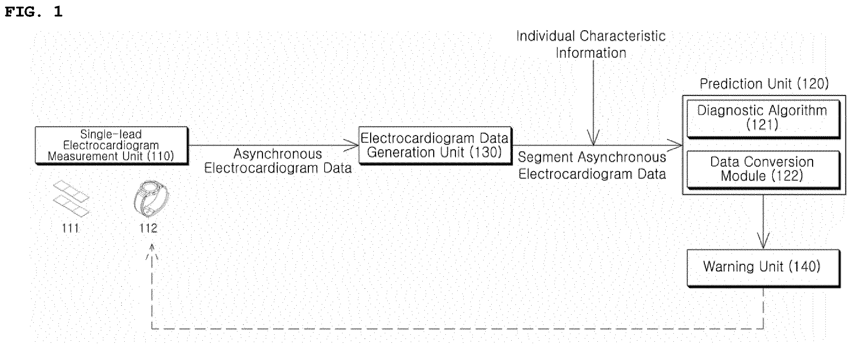

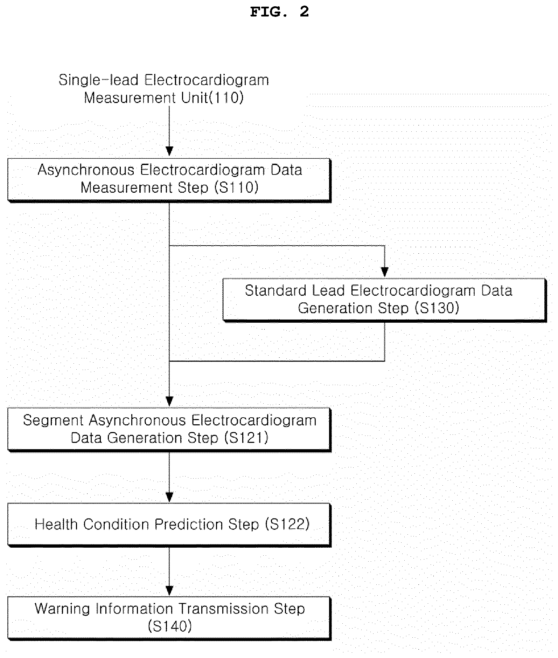

Meanwhile, FIG. 2 is a flowchart showing a prediction method performed by the health condition prediction system using an asynchronous electrocardiogram shown in FIG. 1. Referring to this drawing, a detailed description thereof will be given as follows.

First, asynchronous electrocardiogram data is obtained by measuring electrocardiograms for at least two electrical axes at different times through the single-lead electrocardiogram measurement unit 110 provided with two electrodes by using a method of providing two electrodes and bringing the two electrodes into contact with two portions of the body of an examinee, and is then transmitted to the prediction unit 120 over a short-distance network in step S110.

Thereafter, through the prediction unit 120, segmented asynchronous electrocardiogram data is generated by segmenting the asynchronous electrocardiogram data of the single-lead electrocardiogram measurement unit 110 through the previously trained diagnostic algorithm 121 in step S121, and a health condition is predicted from the segment asynchronous electrocardiogram data in step S122. Through the diagnostic algorithm 121 previously constructed by being trained on a plurality of standard electrocardiogram datasets in which a standard lead electrocardiogram measured and segmented for the same electrical axis as the electrocardiogram previously measured by the single-lead electrocardiogram measurement unit 110 is matched to the presence/absence of a disease and degree of the disease corresponding to the segmented standard lead electrocardiogram, a health condition may be determined by predicting the presence/absence of a disease and the degree of the disease from the asynchronous electrocardiogram data input from the single-lead electrocardiogram measurement unit 110 and then segmented.

Furthermore, through the electrocardiogram data generation unit 130, the individual characteristics of the plurality of pieces of asynchronous electrocardiogram data measured by the single-lead electrocardiogram measurement unit 110 are determined, specific lead electrocardiogram data is identified, and a plurality of pieces of remaining standard lead electrocardiogram data not corresponding to the identified specific lead electrocardiogram data are generated in step S130. Accordingly, the diagnostic algorithm 121 may output and predict the presence/absence of a disease and the degree of the disease by using the asynchronous electrocardiogram data measured by the single-lead electrocardiogram measurement unit 110 and the standard lead electrocardiogram data generated by the electrocardiogram data generation 130 as input or using the standard lead electrocardiogram data generated by the electrocardiogram data generation unit 130 as input.

Thereafter, by means of the warning unit 140, through the single-lead electrocardiogram measurement unit 110 and the prediction unit 120, a health condition is monitored by measuring the reference electrocardiogram of an examinee in his or her usual healthy condition, and whether the electrocardiogram was measured without error and whether the health condition of the examinee is normal are predicted by comparing the electrocardiogram, measured and input in real time by and from the single-lead electrocardiogram measurement unit 110 during daily life, with the reference electrocardiogram. Furthermore, when an error or abnormality is predicted, warning information may be generated and transmitted together with a beep sound through the single-lead electrocardiogram measurement unit 110 in the form of a smartwatch or a separate smart device.

Therefore, through the configuration of the health condition prediction system using an asynchronous electrocardiogram described above, a 2- or more-lead asynchronous electrocardiogram may be easily measured utilizing a single-lead electrocardiogram device or a 6-lead electrocardiogram device. Furthermore, datasets for the training of the prediction model may be generated through the extraction of a 2- or more-lead asynchronous electrocardiogram by segmenting various types of synchronous electrocardiograms accumulated in a medical institution. Accordingly, a disease may be predicted from the 2- or more-lead asynchronous electrocardiogram data measured by the single-lead electrocardiogram device or the 6-lead electrocardiogram device. Furthermore, multi-channel electrocardiogram data is generated from the 2- or more-lead asynchronous electrocardiogram data, so that the presence/absence of a disease and the degree of the disease can be output and predicted with higher accuracy.

The embodiments described in the present specification and the configurations shown in the drawings are only the most preferred embodiments of the present invention and do not represent the overall technical spirit of the present invention. Accordingly, it should be understood that at the time when the present application is filed, there may be various equivalents and modifications that can replace the above embodiments and/or the configurations.

## Claims

1. A health condition prediction system using an asynchronous electrocardiogram, the system comprising:
a single-lead electrocardiogram measurement unit provided with two electrodes, and configured to obtain asynchronous electrocardiogram data by measuring electrocardiograms for at least two electrical axes at different times; and
a prediction unit configured to predict presence/absence of a disease and degree of the disease from the asynchronous electrocardiogram data, input from the single-lead electrocardiogram measurement unit and segmented into specific time units, through a diagnostic algorithm previously constructed by being trained on a plurality of asynchronous segmented standard lead electrocardiogram datasets in which asynchronous segment standard lead electrocardiograms at different times in an asynchronous standard lead electrocardiogram segmented into the specific time units from a synchronous standard lead electrocardiogram measured for a same electrical axis and accumulated in a medical institution server are matched to presence/absence of a disease and degree of the disease corresponding to the asynchronous segment standard lead electrocardiograms.

2. The system of claim 1, further comprising an electrocardiogram data generation unit configured to determine individual characteristics of the plurality of pieces of asynchronous electrocardiogram data measured by the single-lead electrocardiogram measurement unit, identify specific lead electrocardiogram data, and generate a plurality of pieces of remaining standard lead electrocardiogram data not corresponding to the identified specific lead electrocardiogram data;
wherein the diagnostic algorithm outputs and predicts the presence/absence of the disease and the degree of the disease by using the measured asynchronous electrocardiogram data and the generated standard lead electrocardiogram data as input or using the generated standard lead electrocardiogram data as input.

3. The system of claim 3, wherein the electrocardiogram data generation unit generates a plurality of pieces of synchronized standard lead electrocardiogram data.

4. The system of claim 1, wherein the diagnostic algorithm generates the plurality of asynchronous segment standard lead electrocardiogram data sets by asynchronously extracting synchronous segment standard lead electrocardiograms obtained by segmenting standard lead electrocardiogram data, stored in a synchronized form, into specific time units.

5. The system of claim 1, wherein the diagnostic algorithm is constructed to output and predict a presence/absence of a disease and degree of the disease corresponding to standard lead electrocardiogram data by using the standard lead electrocardiogram data, in which time-series information has been deleted from standard lead electrocardiogram data measured synchronously, as input.

6. The system of claim 1, wherein:
the diagnostic algorithm is trained with individual characteristic information of an examinee reflected therein; and
the prediction unit predicts the presence/absence of the disease and the degree of the disease from the asynchronous electrocardiogram data measured by the single-lead electrocardiogram measurement unit with the individual characteristic information reflected therein.

7. The system of claim 6, wherein the individual characteristic information includes demographic information including gender and age of the examinee, basic health information including weight, height and obesity of the examinee, disease-related information including past history, drug history and family history of the examinee, and test information regarding a blood test, genetic test, vital signs, including blood pressure and oxygen saturation, and biosignals of the examinee.
